# EUROPEAN PATENT SPECIFICATION

(11) **EP 1 473 054 B1**
(45) Date of publication and mention of the grant of the patent: **30.08.2006**
(21) Application number: 03252685.7
(22) Date of filing: 28.04.2003
(51) Int. Cl.: A61M 5/36, A61M 5/168

(54) **Dispenser**
Abgabevorrichtung
Distributeur

(43) Date of publication of application: 03.11.2004
(73) Proprietor: The Automation Partnership (Cambridge) Limited, Royston, Hertfordshire SG8 5WY (GB)
(72) Inventor: Bargh, Adrian Neil, London N8 7LS (GB)
(74) Representative: Brunner, Michael John

(56) References cited:
- WO-A-97/14027
- US-A- 3 978 846
- US-A- 5 356 375
- US-A- 2002 059 945
- US-A1- 2002 165 496

## Description

The present invention relates to dispensers for dispensing low volumes of fluids and, more particularly for dispensing small volumes of suspensions.

A conventional syringe pump for dispensing small volumes of fluid is mounted vertically with a three port rotary valve at the top of a syringe chamber and a plunger at the bottom being drawn downwards to draw liquid into the syringe chamber from a reservoir. The aim of mounting a syringe vertically is that it should help to remove air from the syringe as air bubbles rise naturally towards the valve. The valve can be switched to connect the syringe chamber to either an input port or an output port. An inherent problem with the configuration of a three port valve is that there is a dead volume between the syringe chamber and the valve itself. This dead volume results in trapped air being unable to exit the syringe. When the plunger is at the top of its stroke, the air is pushed into the dead volume. However, when the syringe is refilled, because the refilling occurs through the same inlet, the trapped air is pushed back into the syringe ahead of the inflowing fluid. Thus, the configuration of such syringe pump dispensers results in air bubbles becoming trapped and only being persuaded to leave the syringe chamber by manually tapping the syringe. Air bubbles in the syringe can cause the dispensing performance to be reduced to a level at which dispensing reliability is not acceptable, as a result of the fact that the air is much more easily compressed than the liquid to be dispensed, in turn resulting in unacceptable volume variation.

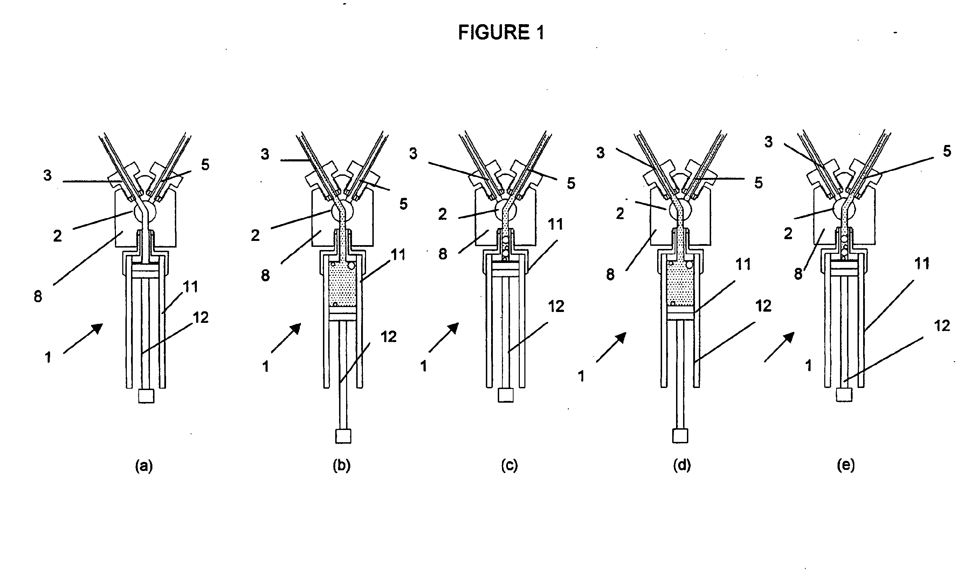

A typical syringe dispenser having a three port valve configuration is shown in Figure 1a - e. These figures show a conventional syringe pump 1 being filled and emptied. Figure 1a shows the syringe pump when it is not in use and the syringe 11 is empty. Figure 1b shows the syringe being filled through the inlet port 3 via the three port valve 2, a number of air bubbles 13 are shown trapped in the syringe 11. Figure 1 c shows the syringe dispensing through the outlet port 5 via the three port valve 2. It shows that once the syringe plunger 12 is at the top of its stroke and the syringe 11 is empty, the air bubbles 13 have moved out into a dead volume 8 that lies between the top of the syringe 11 and the three port valve 2. Because this dead volume 8 it part of the fluid passageway from the inlet port 3 into the syringe 11 then, as shown in Figure 1d, when the syringe is refilled through the inlet port the air bubbles 13 are forced back into the syringe 11 ahead of the incoming fluid 14.

WO 97/14027 discloses a dispensing assembly including a dispenser comprising a vertically disposed syringe pump with separate inlet and outlet ports at the end of the syringe tube opposite the plunger, which are provided with rotary valves.

It has been observed that, as a result of the vertical configuration of the syringe, if a suspension is to be dispensed there can be a considerable difference in concentration between the first volume dispensed and the last within a single syringe stoke as a result of the settling of the suspension towards the position of the syringe plunger.

According to the present invention there is therefore provided a dispensing assembly for dispensing cell suspensions according to claim 1.

The syringe may be arranged to be disposed substantially horizontally resulting in the two two-port rotary valves being positioned one above the other and therefore the inlet valve is positioned below the outlet valve in use.

The syringe plunger may be removable and the end portion of the syringe with a larger cross sectional area. The syringe tube may be a glass tube.

A dispensing array may be formed comprising a plurality of dispensers wherein the dispensing tips and syringe plungers are configured to form substantially parallel arrays.

Such a dispensing array may further comprise an actuator configured to actuate the syringe plungers simultaneously.

The dispensing array may be cleaned and purged of air by the method steps defined in claim 7.

Preferably, the drawing in and dispensing of fluid is repeated, the first fluid being PBS and the second being a water and alcohol mix.

Furthermore there is provided a method of cleaning syringes in a dispensing array according to claim 9.

An example of a dispenser according to the present invention will now be further described with reference to the accompanying drawings in which:
Figures 1a - e shows the configuration and operation of a conventional three port valve syringe pump.
Figures 2a - e shows the sequence of operation and configuration of valves in a dispenser according to the present invention.
Figure 3 shows the configuration of the syringe allowing for the method of cleaning according to the present invention.
Figure 4 shows a dispenser according to the present invention.
Figure 5 shows a dispensing array according to the present invention.

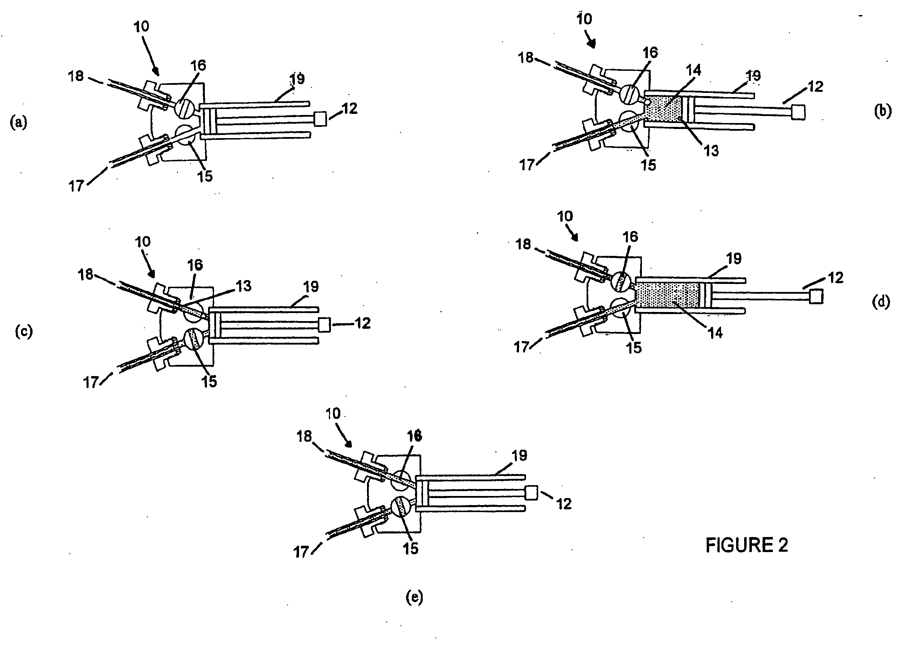

Figures 2a - e show the configuration and operation of an example of a dispenser 10 according to the present invention. The dispenser 10 comprises a syringe 11 with two two-port rotary valves 15 and 16. In use, the syringe 11 is disposed horizontally resulting in the inlet port 17 sloping upwards towards the syringe 11 and the outlet port 18 sloping upwards away from the syringe 11. The syringe 11 consists of a glass barrel 19 and a syringe plunger 12. Figure 2a shows the dispenser 10 when it is not in use. The syringe 11 is devoid of fluid 14. Prior to and following use the dispenser 10 of the present invention is washed through. A two stage washing process is used. Firstly, as shown in Figure 2b, a fluid such as Phosphate Buffered Saline (PBS) is drawn in through the inlet port 17, through the lower of the two-port rotary valves 15 and into the syringe 11. It will be evident from Figure 2b that air bubbles within the fluid may be trapped within the syringe. Valve 15 is then closed and valve 16 opened in order to allow the syringe 11 to be used for dispensing via the upper, outlet, two port valve 16 and the outlet 18. The air bubbles 13 therefore move into the outlet two port valve and leave the syringe 11. These two stages may then be repeated with a water/alcohol mix in order to sterilise the liquid path. Figure 2d shows the syringe filled with the fluid 14 to be dispensed. It is clear that the air bubbles 13 do not re-enter the syringe 11 and therefore do not introduce the inconsistencies in volume dispensed that result from air bubbles, which are compressible, being trapped within the syringe 11. Figure 2e shows the syringe at the end of the priming cycle.

It is apparent from Figure 2 that, in comparison with a conventional three port valve shown in Figure 1, the two two-port valves are separated vertically as a result of the horizontal configuration of the syringe. The horizontal configuration of the syringe 11 also reduces the effect of settling if a suspension is to be dispensed because the height of the column of fluid is shorter and therefore the variation in the concentration of the suspension between the first and last samples dispensed within one stroke of the plunger syringe is reduced by using a dispenser of this type.

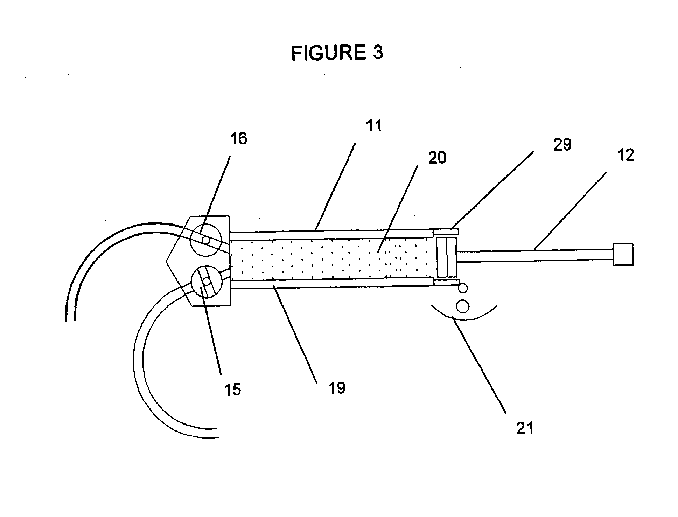

Figure 3 shows detail of the syringe 11. In particular, it shows a method of cleaning the syringe 11. The glass barrel 19 of the syringe 11 has an end portion 29 of thinner glass than the main part of the barrel which allows the syringe plunger 12 to be held securely whilst allowing a small amount of wash fluid 20 to flow past the syringe plunger and out of the end of the glass barrel 19 into an overflow 21. The overflow 21 collects the fluid flowing from the end portion 29 of the syringe 11. This allows the wash fluid 20 to cleanse the inner surface of the glass barrel 19 and the syringe plunger 12 of any crystals that may have formed thereon.

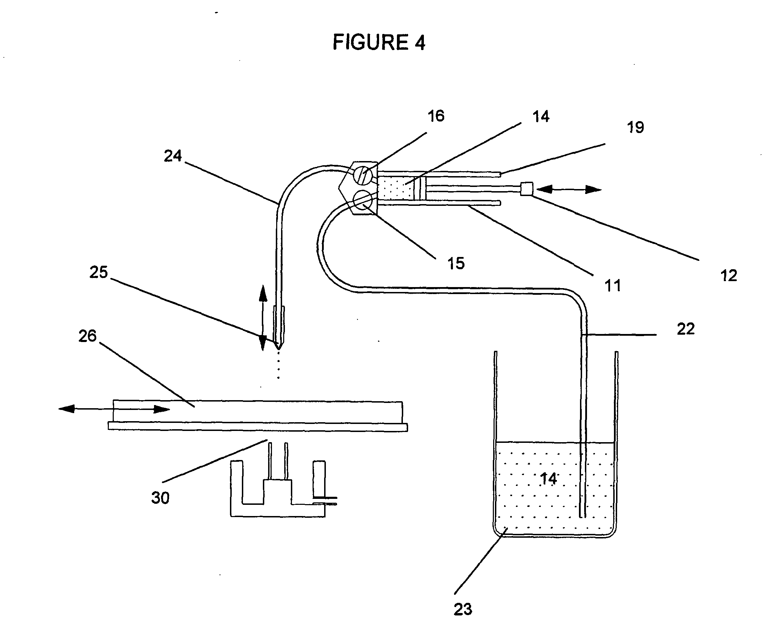

Figure 4 shows further detail of the dispenser. The lower two port valve 15 leads, via liquid input tubing 22, to a liquid reservoir 23 containing the fluid to be dispensed 14. The upper two port valve 16 leads, via liquid output tubing 24, to a dispensing tip 25 from which fluid 14 is dispensed on to a conventional micro plate 26. When the syringe is washed with PBS or alcohol/water mix the dispensing tip 25 is repositioned such that the wash fluids are dispensed into a gutter 30 positioned below the micro plate 26. The micro plate 26 is capable of horizontal movement to allow fluid to be dispensed to different parts of the micro plate 26 and also to allow access to the gutter 30 which is positioned below the dispensing tip 25. When the dispenser 10 is washed the dispensing tip 25 can be lowered into the gutter 30. The gutter 30 has substantially parallel walls that conform closely to the outer surface of the dispensing tip 25. As the PBS or water/alcohol mixture is dispensed through the dispensing tip 25 the fluid is forced to flow up around the outer surface of the dispensing tip 25 thereby cleansing the tip 25.

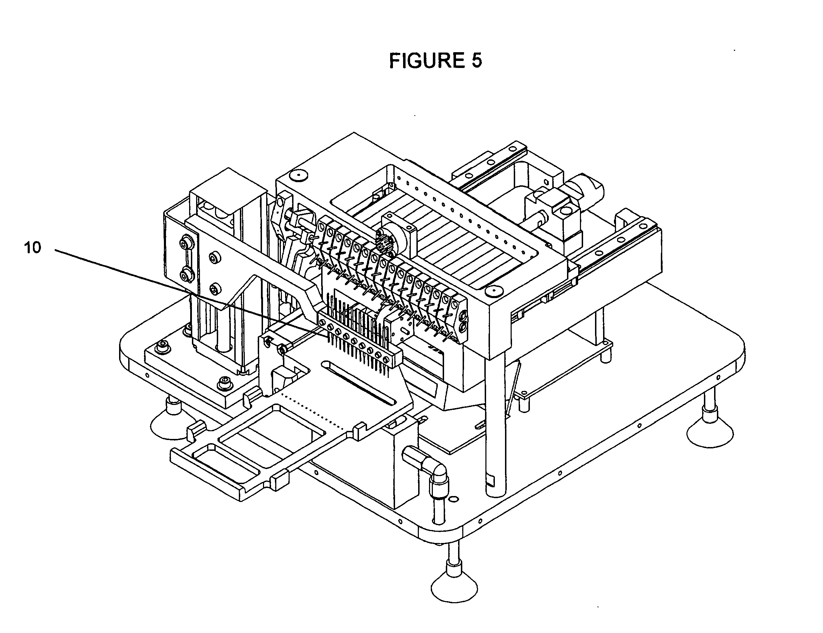

A dispensing array can be formed from a plurality of dispensers 10 as shown in Figure 5. The dispensing tips 25 of the dispensers are formed into a substantially parallel dispensing array. Furthermore, the syringes 11 are also formed into a substantially parallel array such that the plungers 12 of the dispensers can be actuated simultaneously by an actuator moving in a plane perpendicular to the plane of the array of plungers 12.

## Claims

1. A dispensing assembly (10) for dispensing cell suspensions, the assembly (10) including a dispenser comprising:
a dispensing tip (25);
a syringe (11) having a tube, a movable plunger (12), an inlet port (17) and an outlet port (18), and **characterised by** the syringe (11) being arranged substantially horizontally, in use,
two two-port rotary valves (15, 16) providing an inlet valve and an outlet valve, at the end of the tube opposite the plunger, and connected respectively, to the inlet port (17) and the outlet port (18);
the inlet port (17) being positioned below the outlet port (18) and being connectable through the inlet valve (15), in use, to a reservoir (23) of fluid to allow fluid to be drawn into the syringe (11) from the reservoir (23), and the outlet port (18)being connectable, in use, through the outlet valve (16) to the dispensing tip (25) to allow fluid to be dispensed therefrom.

2. A dispensing assembly (10) according to claim 1, wherein the syringe plunger (12) is removable.

3. A dispensing assembly (10) according to any one of the preceding claims, further comprising an end portion of the syringe (11) with a larger cross sectional area.

4. A dispensing assembly (10) according to any one of the preceding claims wherein the syringe tube is a glass tube (19).

5. A dispensing array comprising a plurality of dispensing assemblies (10) according to any one of claims 1 to 4, wherein the dispensing tips (25) and syringe plungers (12) are configured to form substantially parallel arrays.

6. A dispensing array according to claim 5, further comprising an actuator configured to actuate the syringe plungers (11) simultaneously.

7. A method of cleaning and purging air from a dispensing array comprising a plurality of dispensing assemblies according to any one of claims 1-4, wherein the dispensing tips (25) and syringe plungers (12) are configured to form substantially parallel arrays, the method comprising steps of:
providing said dispensing array
drawing fluid through the lower two-port rotary valve (15) into the syringe (11),
lowering the array of dispensing tips (25) into the gutter (30),
depressing the plunger (12) to force the liquid out through the upper two-port rotary valve (16), forcing any trapped air bubbles into the upper two-port rotary valve and forcing the fluid through the tips (25) such that it flows up around the tip end.

8. A method according to claim 7, wherein the drawing in and dispensing of fluid is repeated, the first fluid being PBS and the second being a water and alcohol mix.

9. A method of cleaning syringes in a dispensing array comprising a plurality of dispensing assemblies according to any one of claims 1-4, (10) wherein the dispensing tips (25) and syringe plungers (12) are configured to form substantially parallel arrays, the method comprising the steps of:
providing said dispensing array
drawing fluid through the lower two port valve (15) until the syringe (11) is full,
continuing to draw fluid forcing the plunger (12) to be disconnected from the syringe tube and allowing fluid to flow out of the end of the tube (19) and into the overflow zone.

## Patentansprüche

1. Spendeanordnung (10) zum Abgeben von Zellsuspensionen, wobei die einen Spender enthaltende Anordnung (10) umfasst:
eine Spendespitze (25);
eine Spritze (11) mit einem Rohr, einem beweglichen Kolben (12), einer Einlassöffnung (17) und einer Auslassöffnung (18)
**dadurch gekennzeichnet, dass**
die Spritze (11) im Gebrauch im Wesentlichen horizontal angeordnet ist;
zwei Zweiweg-Drehschieber (15, 16), die ein Einlassventil und ein Auslassventil an dem dem Kolben entgegengesetzten Ende des Rohrs darstellen, und die jeweils mit der Einlassöffnung (17) und der Auslassöffnung (18) verbunden sind;
die Einlassöffnung (17), die unter der Auslassöffnung (18) angeordnet ist und beim Gebrauch über das Einlassventil (15) mit einem Vorratsbehälter (23) für Fluid so verbunden werden kann, dass Fluid aus dem Vorratsbehälter (23) in die Spritze (11) gesaugt werden kann, und die Auslassöffnung (18) im Gebrauch über das Auslassventil (16) mit der Spendespitze (25) verbunden werden kann, um daraus ein Fluid abzugeben.

2. Spendeanordnung (10) nach Anspruch 1, bei der der Spritzenkolben (12) entfernt werden kann.

3. Spendeanordnung (10) nach einem der vorhergehenden Ansprüche, die ferner einen Endabschnitt der Spritze (11) mit einer größeren Querschnittsfläche umfasst.

4. Spendeanordnung (10) nach einem der vorhergehenden Ansprüche, bei der das Rohr der Spritze ein Glasrohr (19) ist.

5. Spendegruppe, die eine Vielzahl von Spendeanordnungen (10) nach einem der Ansprüche 1 bis 4 umfasst, bei der die Spendespitzen (25) und die Spritzenkolben (12) so gestaltet sind, dass sie im Wesentlichen parallele Anordnungen ausbilden.

6. Spendegruppe nach Anspruch 5, die des Weiteren ein Stellglied umfasst, das so gestaltet ist, dass es die Kolben der Spritzen (11) gleichzeitig betätigt.

7. Verfahren zum Reinigen und Blasen von Luft aus einer Spendegruppe, die eine Vielzahl von Spendeanordnungen nach einem der Ansprüche 1 bis 4 umfasst, in der die Spendespitzen (25) und die Kolben (12) der Spritze so gestaltet sind, dass sie im Wesentlichen parallele Anordnungen ausbilden, wobei das Verfahren die Schritte umfasst:
Bereitstellen der Spendegruppe,
Saugen von Fluid durch den unteren Zweiweg-Drehschieber (15) in die Spritze (11),
Absenken der Gruppe von Spendespitzen (25) in die Ablaufrinne (30),
Niederdrücken des Kolbens (12), um das Fluid durch den oberen Zweiweg-Drehschieber (16) herauszupressen, alle eingeschlossenen Luftblasen in den oberen Zweiweg-Drehschieber zu befördern und das Fluid so durch die Spitzen (25) zu drücken, dass sie um das Ende der Spitze herum abfließt.

8. Verfahren nach Anspruch 7, in dem das Einsaugen und Spenden von Fluid wiederholt wird, wobei das erste Fluid PBS ist und das zweite eine Mischung aus Wasser und Alkohol ist.

9. Verfahren zum Reinigen von Spritzen in einer Spendegruppe, die eine Vielzahl von Spendeanordnungen (10) nach einem der Ansprüche 1 bis 4 umfasst, in der die Spendespitzen (25) und die Kolben (12) der Spritze so gestaltet sind, dass sie im Wesentliche parallele Anordnungen ausbilden, wobei das Verfahren die Schritte umfasst:
Bereitstellen der Spendegruppe,
Saugen von Fluid durch den unteren Zweiweg-Drehschieber (15) bis die Spritze (11) voll ist,
weiteres Einsaugen von Fluid, wodurch der Kolben (12) gezwungen wird, sich von dem Rohr der Spritze zu lösen, und das Fluid aus dem Ende des Rohrs (19) und in den Überlaufbereich hinein fließen kann.

## Revendications

1. Assemblage dispensateur (10) pour la distribution de suspension cellulaires, l'assemblage (10) incluant un dispensateur comprenant :
une pointe dispensatrice (25) ;
une seringue (11) ayant un tube, un piston mobile (12), un orifice d'entrée (17) et un orifice de sortie (18) et **caractérisé par le fait que** la seringue (11) est disposée sensiblement horizontalement lors de l'utilisation,
deux robinets à boisseau tournant, à deux orifices (15, 16), procurant un robinet d'entrée et un robinet de sortie, à l'extrémité du tube, opposée au piston, et connectés respectivement à l'orifice d'entrée (17) et à l'orifice de sortie (18) ;
l'orifice d'entrée (17) étant positionné sous l'orifice de sortie (18) et pouvant être connecté par le robinet d'entrée (15), lors de l'utilisation, à un réservoir (23) de fluide pour permettre au fluide d'être tiré dans la seringue (11) depuis le réservoir (23), et l'orifice de sortie (18) pouvant être connecté par le robinet de sortie (16), lors de l'utilisation, à la pointe dispensatrice (25) pour permettre au fluide d'être distribué par celle-ci.

2. Assemblage dispensateur (10) selon la revendication 1, dans lequel le piston (12) de la seringue est amovible.

3. Assemblage dispensateur (10) selon l'une quelconque des revendications précédentes, comprenant en outre une partie terminale de la seringue (11) avec une coupe transversale plus grande.

4. Assemblage dispensateur (10) selon l'une quelconque des revendications précédentes, dans lequel le tube de la seringue est un tube en verre (19).

5. Zone dispensatrice comprenant une série d'assemblages dispensateurs (10) selon l'une quelconque des revendications 1 à 4, dans laquelle les pointes dispensatrices (25) et les pistons de seringue (125) sont configurés pour former des zones sensiblement parallèles.

6. Zone dispensatrice selon la revendication 5, comprenant en outre un appareil de commande configuré pour actionner les pistons de seringue (11), simultanément.

7. Procédé de nettoyage et de purge de l'air d'une zone dispensatrice, comprenant une série d'assemblages dispensateurs (10) selon l'une quelconque des revendications 1 à 4, dans lequel les pointes dispensatrices (25) et les pistons de seringue (125) sont configurés pour former des zones sensiblement parallèles, le procédé comprenant les étapes de :
disposer ladite zone dispensatrice,
aspirer le fluide par le robinet à boisseau tournant à deux orifices, inférieur (15) dans la seringue (11),
abaisser la zone des pointes dispensatrices (25) dans la gouttière (30),
presser le piston (12) pour forcer le liquide à sortir par le robinet à boisseau tournant à deux orifices, supérieur (16), forçant toute bulle piégée dans le robinet à boisseau tournant à deux orifices, inférieur et forçant le fluide au travers des points (25) de sorte qu'il s'écoule autour de l'extrémité en pointe.

8. Procédé selon la revendication 7, dans lequel l'aspiration et la distribution du fluide sont répétés, le premier fluide étant du PBSZ et le deuxième étant un mélange d'eau et d'alcool.

9. Procédé pour nettoyer des seringues dans une zone dispensatrice, comprenant une série d'assemblages dispensateurs (10) selon l'une quelconque des revendications 1 à 4, dans lequel les pointes dispensatrices (25) et les pistons de seringue (125) sont configurés pour former des zones sensiblement parallèles, le procédé comprenant les étapes de :
disposer ladite zone dispensatrice,
aspirer le fluide par le robinet à boisseau tournant à deux orifices, inférieur (15) jusqu'à ce que la seringue (11) soit pleine,
continuer à aspirer le fluide pour forcer le piston (12) à être déconnecté du tube de la seringue et laisser le fluide s'écouler de l'extrémité du tube (19) et dans la zone de trop-plein.
